# EUROPÄISCHE PATENTSCHRIFT

(11) **EP 0 505 514 B1**
(45) Veröffentlichungstag und Bekanntmachung des Hinweises auf die Patenterteilung: **22.05.1996**
(21) Anmeldenummer: 91913362.9
(22) Anmeldetag: 01.08.1991
(51) Int. Cl.: C12P 17/18

(54) **VERFAHREN ZUR HERSTELLUNG VON $g(b)-CARBOLIN-DERIVATEN**
PROCESS FOR PRODUCING $g(b)-CARBOLINE DERIVATES
PROCEDE DE PRODUCTION DE DERIVES DE $g(b)-CARBOLINE

(30) Priorität: 13.09.1990 DE 4029389
(43) Veröffentlichungstag der Anmeldung: 30.09.1992
(73) Patentinhaber: SCHERING AKTIENGESELLSCHAFT, D-13342 Berlin (DE)
(72) Erfinder: WEBER, Alfred, Dr., D-1000 Berlin 37 (DE); KENNECKE, Mario, Dr., D-1000 Berlin 33 (DE); HILSCHER, Jean-Claude, Dr., D-1000 Berlin 33 (DE); NICKISCH, Klaus, Dr., D-1000 Berlin 49 (DE)
(86) Internationale Anmeldenummer: DE9100626
(87) Internationale Veröffentlichungsnummer: WO9205269

(56) Entgegenhaltungen:
- EP-A- 130 140
- EP-A- 234 173

## Beschreibung

Die Erfindung betrifft ein Verfahren zur Herstellung von β-Carbolin-Derivaten der allgemeinen Formel I 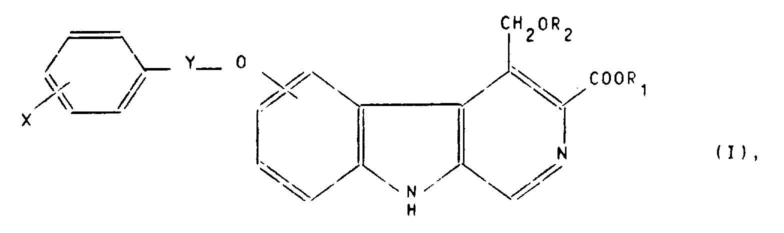 worin
- X: ein Wasserstoffatom oder ein Halogenatom,
- Y: eine Kohlenstoff-Sauerstoff-Bindung oder eine Methylengruppe,
- R₁: eine Alkylgruppe mit bis zu 6 Kohlenstoffatomen und
- R₂: eine Alkylgruppe mit bis zu 4 Kohlenstoffatomen darstellen aus 1,2,3,4-Tetrahydro-β-carbolin-Derivaten der allgemeinen Formel II 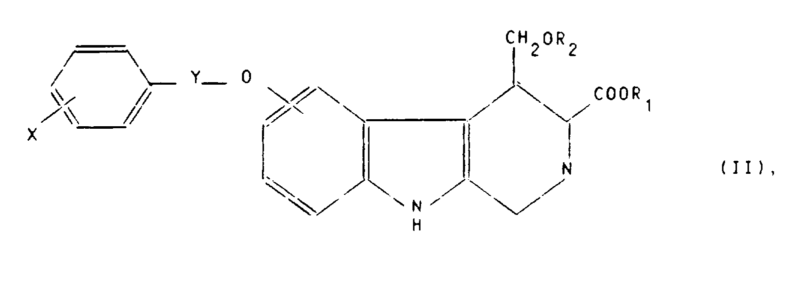
worin
X,Y,R₁ und R₂ die oben genannte Bedeutung besitzen.

β-Carbolin-Derivate der allgemeinen Formel I sind bekanntlich pharmakologisch wirksame Substanzen, welche beispielsweise durch chemische Dehydrierung von 1,2,3,4-Tetrahydro-β-carbolin-Derivaten der allgemeinen Formel II hergestellt werden können. (EP-A 0130140, EP-A 0234173 und EP-A 0239667). Diese chemische Dehydrierung ist aber recht aufwendig und die hierbei erzielbaren Ausbeuten sind gering.

Es wurde nun gefunden, daß man die Dehydrierung der 1,2,3,4-Tetrahydro-β-carbolin-Derivate der allgemeinen Formel II in einfacher Weise, unter umweltfreundlichen Bedingungen und unter Erzielung befriedigender Ausbeuten durchführen kann, indem man diese Verbindungen mit einer Pilzkultur der Gattungen Fusarium oder Myrothecium fermentiert. Nach den bisher vorliegenden Untersuchungen scheinen Pilzkulturen der Spezies Myrothecium verrucaria zur Durchführung des erfindungsgemäßen Verfahrens besonders geeignet zu sein.

Es ist für den Fachmann sehr überraschend, daß man die 1,2,3,4-Tetrahydro-β-carbolin-Derivate der allgemeinen Formel I mit Hilfe des erfindungsgemäßen Verfahrens in die β-Carbolin-Derivate der allgemeinen Formel umwandeln kann, denn derartige Dehydrierung an Heteroaromaten sind nicht vorbeschrieben. Uberraschend ist auch die Tatsache, daß bei dieser Fermentation die Estergruppen der Substrate nicht gespalten werden.

Das erfindungsgemäße Verfahren wird unter den gleichen Fermentationsbedingungen durchgeführt, welche man auch bei den bekannten mikrobiologischen Umwandlungen von Substraten mit Pilzkulturen anwendet.

Unter den für Pilzkulturen üblicherweise verwendeten Kulturbedingungen werden in einem geeigneten Nährmedium unter Belüften Submerskulturen angezüchtet. Dann setzt man den Kulturen das Substrat (in einem geeigneten Lösungsmittel gelöst oder in emulgierter Form) zu und fermentiert, bis eine maximale Substratumwandlung erreicht ist.

Geeignete Substratlösungsmittel sind beispielsweise Methanol, Ethanol, Glykolmonomethylether, Dimethylformamid oder Dimethylsulfoxyd. Die Emulgierung des Substrats kann beispielsweise bewirkt werden, indem man dieses in mikronisierter Form oder in einem mit Wasser mischbaren Lösungsmittel (wie Methanol, Ethanol, Aceton, Glykolmonomethylether, Dimethylformamid oder Dimethylsulfoxyd) gelöst unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispiel Ethylenoxyaddukte oder Fettsäureester von Polyglykolen. Als geeignete Emulgatoren seien die handelsüblichen Netzmittel Tegin®, Tween® und Span® beispielsmäßig genannt.

Die optimale Substratkonzentration, Substratzugabezeit und Fermentationsdauer ist von der Art des verwendeten Substrates und Mikroorganismus und den Fermentationsbedingungen abhängig. Diese Größen müssen, wie dies bei mikrobiologischen Steroidumwandlungen allgemein erforderlich ist, im Einzelfall durch Vorversuche, wie sie dem Fachmann geläufig sind, ermittelt werden.

Die als Ausgangsmaterial für das erfindungsgemäße Verfahren verwendeten 1,2,3,4-Tetrahydro-β-carbolin-Derivate der allgemeinen Formel II können als Substituenten X beispielsweise ein Wasserstoffatom oder ein vorzugsweise in p-Stellung befindliches Chloratom tragen. Geeignete Substituenten R₁ des Substrates sind beispielsweise die Methylgruppe, die Ethylgruppe, die Propylgruppe, die Isopropylgruppe oder die tert.-Butylgruppe. Als Substituent R₂ der Substrate seien beispielsweise die Ethylgruppe, die Propylgruppe, die Isopropylgruppe und insbesondere die Methylgruppe genannt. Diese Verbindungen sind bekannt, oder können analog zu dem Verfahren hergestellt werden das in der EP-A 0130140 beschrieben ist.

Das nachfolgende Ausführungsbeispiel dient zur näheren Erläuterung des erfindungsgemäßen Verfahrens.

### Beispiel

a) Ein 2 1 Erlenmeyer mit 500 ml sterilem Nährmedium enthaltend
   - 3 %: Glucose
   - 1 %: Cornsteep liquor
   - 0,2 %: NaNO₃
   - 0,1 %: KH₂PO₄
   - 0,05 %: KCl
   - 0,002 %: FeSO₄
   - 0,2 %: K₂HPO₄
   mit einem pH von 6,1 wird mit 5 ml einer Suspension einer Myrothecium verrucaria DSM 2087 Kultur beimpft und 48 Stunden bei 30° C mit 180 Umdrehungen pro Minute geschüttelt.
b) 20 Erlenmeyer (500 ml) mit jeweils 100 ml sterilem Nährmedium enthaltend
   - 1,0 %: Cornsteep liquor
   - 1,25 %: Sojapuder
   - eingestellt auf pH 6,2 -
   werden mit jeweils 10 ml der Myrothecium-verrucaria-Anzuchtskultur beimpft und Stunden auf einem Rotationsschüttler mit 180 Umdrehungen pro Minute bei 30° C inkubiert.
   Dann setzt man jeder Kultur 0,04 g in 1 ml Dimethylformamid gelöstes und sterilfiltrierte 6-Benzyloxy-4-methoxymethyl-1,2,3,4-tetrahydro-β-carbolin-isopropylester zu und fermentiert weitere 113 Stunden.
c) Die vereinigten Kulturen werden mit Methylisobutylketon extrahiert und der Extrakt im Rotationsverdampfer bei max. 50° C unter Vakuum eingeengt. Anschließend erfolgt eine Reinigung durch Chromatographie über eine Kieselgelsäule.

Man erhält 0,5 g 6-Benzyloxy-4-methoxymethyl-β-carbolin-3-carbonsäureisopropylester vom Schmelzpunkt 150 - 151° C.

## Patentansprüche

1. Verfahren zur Herstellung von β-Carbolin-Derivaten der allgemeinen Formel I 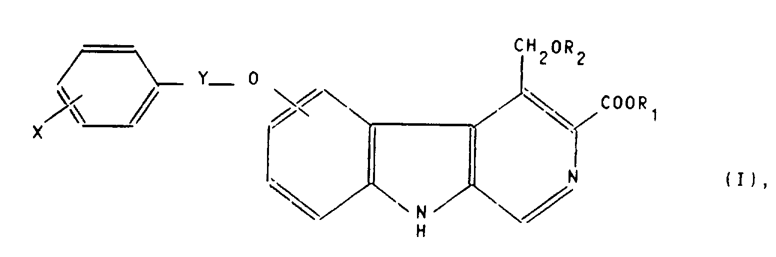 worin
X ein Wasserstoffatom oder ein Halogenatom,
Y eine Kohlenstoff-Sauerstoff-Bindung oder eine Methylengruppe,
R₁ eine Alkylgruppe mit bis zu 6 Kohlenstoffatomen und
R₂ eine Alkylgruppe mit bis zu 4 Kohlenstoffatomen darstellen, aus 1,2,3,4-Tetrahydro-β-carbolin-Derivaten der allgemeinen Formel II 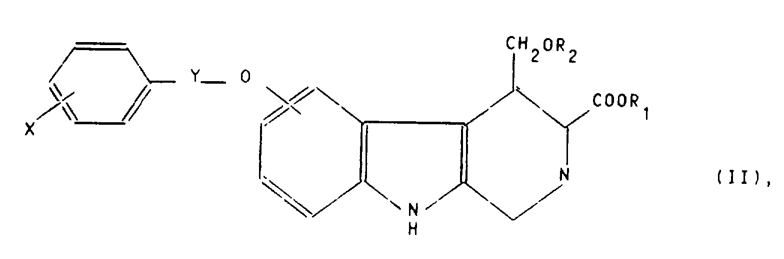
worin
X,Y,R₁ und R₂ die oben genannte Bedeutung besitzen,
dadurch gekennzeichnet, daß man die 1,2,3,4-Tetrahydro-β-carbolin-Derivate mit einer Pilzkultur der Gattung Fusarium oder Myrothecium fermentiert.

2. Verfahren zur Herstellung von β-Carbolin-Derivaten der allgemeinen Formel I gemäß Patentanspruch 1,
dadurch gekennzeichnet, daß man die Fermentation mit einem Pilzstamm der Spezies Myrothecium verrucaria durchführt.

## Claims

1. Process for the preparation of β-carboline derivatives of the general formula I 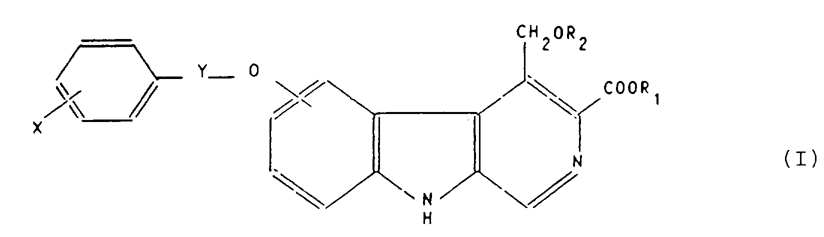 wherein
X is a hydrogen atom or a halogen atom,
Y is a carbon-oxygen bond or a methylene group,
R₁ is an alkyl group having up to 6 carbon atoms and
R₂ is an alkyl group having up to 4 carbon atoms,
from 1,2,3,4-tetrahydro-β-carboline derivatives of the general formula II 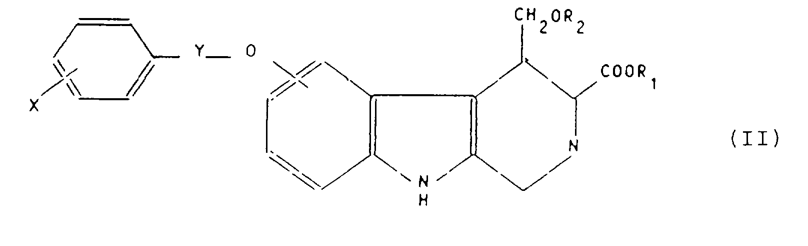 wherein
X, Y, R₁ and R₂ have the meanings given above,
characterised in that the 1,2,3,4-tetrahydro-β-carboline derivatives are fermented with a fungus culture of the genus Fusarium or Myrothecium.

2. Process for the preparation of β-carboline derivatives of the general formula I according to patent claim 1, characterised in that the fermentation is carried out with a fungus strain of the species Myrothecium verrucaria.

## Revendications

1. Procédé pour la préparation de dérivés de la β-carboline de formule générale I 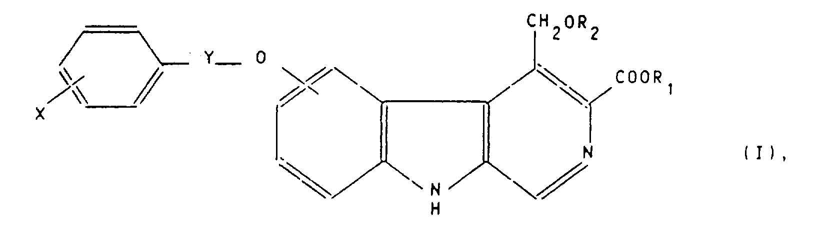 dans laquelle
X représente un atome d'hydrogène ou un atome de d'halogène,
Y représente une liaison carbone-oxygène ou un groupe méthylène,
R₁ représente un groupe alkyle avec jusqu'à 6 atomes de carbone, et
R₂ représente un groupe alkyle avec jusqu'à 4 atomes de carbone
à partir de dérivés de la 1,2,3,4-tétrahydro-β-carboline de formule générale II 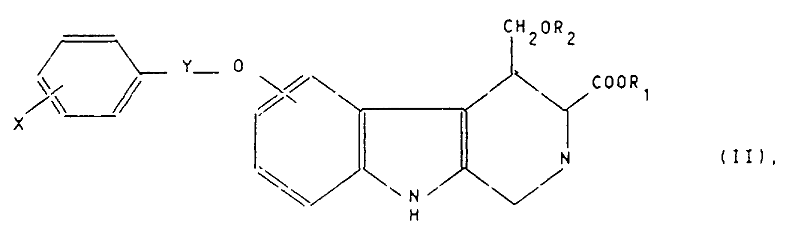 dans laquelle
X, Y, R₁ et R₂ ont la signification donnée ci-dessus, caractérisé en ce qu'on fermente les dérivés de la 1,2,3,4-tétrahydro-β-carboline avec une culture de champignons du genre Fusarium ou Myrothecium.

2. Procédé pour la préparation de dérivés de la β-carboline de formule générale I selon la revendication 1, caractérisé en ce qu'on met en oeuvre la fermentation à l'aide d'une souche de champignons de l'espèce Myrothecium verrucaria.
